# EUROPEAN PATENT SPECIFICATION

(11) **EP 4 273 216 B1**
(45) Date of publication and mention of the grant of the patent: **03.04.2024**
(21) Application number: 22171925.5
(22) Date of filing: 05.05.2022
(51) Int. Cl.: C12M 1/34, E21B 49/08

(54) **HIGH-PRESSURE CONTAINER FOR TAKING A RAW MATERIAL SAMPLE CONTAINING OXYGEN-SENSITIVE MICROORGANISMS FROM A DEPOSIT AND METHOD FOR TAKING SUCH A RAW MATERIAL SAMPLE**
HOCHDRUCKBEHÄLTER ZUR ENTNAHME EIENR ROHSTOFFPROBE, DIE SAUERSTOFFEMPFINDLICHE MIKROORGANISMEN UMFASST, AUS EINER LAGERSTÄTTE UND VERFAHREN ZUR ENTNAHME EINER SOLCHEN ROHSTOFFPROBE
RÉCIPIENT À HAUTE PRESSION POUR LE PRÉLÈVEMENT D'UN ÉCHANTILLON DE MATIÈRE PREMIÈRE CONTENANT DES MICRO-ORGANISMES SENSIBLES À L'OXYGÈNE DANS UN DÉPÔT ET MÉTHODE POUR LE PRÉLÈVEMENT D'UN TEL ÉCHANTILLON DE MATIÈRE PREMIÈRE

(43) Date of publication of application: 08.11.2023
(73) Proprietor: Microbify GmbH, 94315 Straubing (DE)
(72) Inventor: Dr. Schmid, Georg, 93053 Regensburg (DE); Dengler, Linda, 93109 Wiesent (DE); Böllmann, Andrea, 93053 Regensburg (DE); Kaul, Anja, 93053 Regensburg (DE)
(74) Representative: Aera A/S

(56) References cited:
- US-A1- 2012 070 883
- PATRICK SAUER ET AL: "A System for Incubations at High Gas Partial Pressure", FRONTIERS IN MICROBIOLOGY, vol. 3, 1 January 2012 (2012-01-01), XP055187646, ISSN: 1664-302X, DOI: 10.3389/fmicb.2012.00025
- R. JOHN PARKES ET AL: "Culturable prokaryotic diversity of deep, gas hydrate sediments: first use of a continuous high-pressure, anaerobic, enrichment and isolation system for subseafloor sediments (DeepIsoBUG)", ENVIRONMENTAL MICROBIOLOGY, vol. 11, no. 12, 1 December 2009 (2009-12-01), pages 3140-3153, XP55238697, GB ISSN: 1462-2912, DOI: 10.1111/j.1462-2920.2009.02018.x

## Description

The present invention concerns a high-pressure container for taking a raw material sample containing oxygen-sensitive, such as anaerobic, microorganisms from a deposit and a method for taking such a raw material sample, in detail according to the independent claims.

Anaerobic microorganisms (bacteria as well as archaea) are microorganisms that live in the absence of molecular (atmospheric) oxygen. Molecular oxygen is harmful to these organisms and leads to the rapid death of the cells. Such microorganisms can live in (underground) deposits or reservoirs, such as (natural) gas storage facilities and in the associated infrastructure, such as pipelines, valves and gas treatment plants. Anaerobic microorganisms can live in this infrastructure and in the worst case may damage it, e.g. by forming corrosive substances. On the other hand, microorganisms can also be used to produce "green natural gas" in former natural gas deposits such as cavities or pores. In both cases, it is therefore of interest to determine whether anaerobic microorganisms live in the deposit or its associated infrastructure.

To prove their presence, the microorganisms must therefore first be taken undamaged from the deposit in order to subsequently examine them e.g. in the laboratory. On the one hand, no contamination by foreign germs may occur at the sampling point. Secondly, the samples must not come into contact with molecular oxygen from the atmosphere of air, as this would kill anaerobic microorganisms. In addition, sampling is made more difficult by the fact that a relatively high pressure of up to 200 bar or more is usually present especially in underground deposits and the associated infrastructure.

Patrick Sauer et al. ("A System for Incubations at High Gas Partial Pressure", Frontiers in Microbiology, vol. 3, 1 January 2012) describe a high-pressure high-temperature incubation system which is a moderately priced alternative to existing systems and which is easy to construct and to handle. The system is suitable for a wide range of applications in geo- and bio-sciences. The system Sauer et al. allows the incubations at elevated pressure and temperature conditions (up to 120 °C and 60MPa) as well as manipulating the dissolved gases throughout the experiment.

Up to now, such samples are taken in a commercially available glass flask. The flask is evacuated from air and flushed with an inert gas like nitrogen or argon and is closed with an airtight stopper. In addition, the flask may be autoclaved before, to sterilize it. For sampling, the flask is opened and held under the tap at the sampling point, which tap is then opened so that the sample runs into the flask. The flask is filled to the brim, then the airtight stopper is used to reseal the flask. However, when the sample is filled into the flask, the inert gas escapes and the sample is for a short but significant time exposed to air. This can be seen directly, since often the contents of the bottle quickly become cloudy. Thereby, the turbidity may be caused by metal oxides which are formed by the reaction of metal ions with atmospheric oxygen and precipitate in the solution. This turbidity is therefore an indication that the sample has reacted with the atmospheric oxygen and that a large proportion of the microorganisms in the sample may have already died. This effect may cause a decrease of the detected microorganisms in the sample or may need a greater effort for the detection.

It is therefore the object of the present invention to provide a high-pressure container and a method for taking a raw material sample containing oxygen-sensitive, such as anaerobic, microorganisms from a deposit that are improved as compared to the devices or methods known from the state of the art. In detail, the inventive high-pressure container as well as the sampling method shall provide a more reliable detection for anaerobic microorganisms, increase the survival rate of the sampled microorganisms as well as lower the effort for sampling and reproduction of the microorganisms after taking the sample and examining it in the laboratory.

The object is solved by a high-pressure container and a method for taking a raw material sample containing oxygen-sensitive, such as anaerobic, microorganisms from a deposit according to the independent claims. The dependent claims describe particularly useful embodiments of the invention.

According to the invention, a high-pressure container for taking a raw material sample containing oxygen-sensitive, such as anaerobic, microorganisms from a deposit, comprises an inlet for feeding the raw material sample under pressure into a sample chamber of the high-pressure container and an outlet for discharging the raw material sample therefrom, the inlet being connectable to the deposit by means of a feed line and a directional control valve being assigned to the feed line - as seen in the direction of flow of the raw material sample into the sample chamber upstream of the inlet - by means of which the feed line can be selectively connected on the one hand to an outlet for the raw material sample, bypassing the inlet, in order to flush the feed line with the raw material sample and on the other hand the feed line can be connected to the inlet in a flow-conducting manner in order to feed the raw material sample from the feed line via the inlet to the sample chamber, wherein the high-pressure container (1) comprises a sample container (12) and a lid (13) which can be placed thereon, which together delimit or form the sample chamber (4), wherein the lid (13) forms the inlet (3) and the outlet (5) for the raw material sample and the feed line (6) and the directional control valve (7) are fastened or can be fastened to the lid (13) and wherein the directional control valve (7) is designed as a three-way valve, which being switchable between three positions, namely a first position, in which the raw material from the deposit (2) being guided under pressure to the discharge (8), a second position, in which the raw material from the deposit (2) being guided under pressure to the sample chamber (4) and a third position, in which the raw material being trapped in the sample chamber (4) by cutting of both return connections of the raw material sample to the discharge (8) or the deposit (2).

The directional control valve can have another position in which it closes the sample chamber against the escape of the sample material through the port connected to the inlet and the discharge, keeping the collected raw material sample in the sample chamber. Thus the directional control valve is designed as a three-way valve, and being switchable between three different positions, namely, a first position, in which the raw material from the deposit may be guided to the discharge, a second position, in which the raw material sample may be guided to the sample chamber and a third position, in which the raw material sample may be trapped in the sample chamber by closing both return connections of the raw material sample to the discharge or the deposit. Therefore, the directional control valve may comprise a corresponding valve spool which is switchable between these three positions, in which the valve spool restricts or permits flow and thus it controls the fluid flow in the said connections.

Comparative tests have shown that with the inventive high-pressure container as well as the sampling method, samples can be taken in which a higher number of microorganisms is still living and the sample is less cloudy than taken with a conventional glass flask.

The present invention thus allows the sampling of living oxygen-sensitive, such as anaerobic, microorganisms from the deposit, like an underground deposit or reservoir or an infrastructure coupled therewith. The detection of the microorganisms as well as the survival rate of the sampled microorganisms may be enhanced by first anaerobic sample taking and second by pressure equalisation through slow pressure reduction in the sample chamber. The anaerobic sampling may be technically achieved by flushing the feed line through with the sample to take and leading it first to the discharge by respective actuating the directional control valve and only after flushing the feed line, the sample is conducted into the sample chamber of the high-pressure container. Thus, the feed line is purged with the material sample itself such that possible impurities and atmospheric oxygen are removed from the feed line, even before they enter the sample chamber. Further, the anaerobic sampling can be enhanced by evacuating the sample chamber of the high-pressure container from molecular oxygen containing air, filling it with inert gas and/or sterilizing it in an autoclave before filling it with the raw sample material. The pressure equalisation in the inner of the sample chamber may be technically achieved by selective and slow decompression of the sample chamber of the high-pressure container already filled with the raw material sample, especially by a shut-off valve that is associated with the outlet and is connected downstream of the outlet in the direction of flow of the raw material sample, as will be described further below in more detail. Thus, in the inner part of the high-pressure container, after sample taking, nearly or even the same pressure exists as in the deposit. So, the whole container can be taken to e.g. laboratory with having the pressure of the deposit established until the sample is examined. Furthermore, the sampling can be enhanced if the whole high-pressure container is able to be disinfected e.g. in an autoclave.

Further, the inventive high-pressure container is foreseen to withstand pressures in its inner sample chamber of 10 to 200 bar. It therefore can be designed from steel, especially stainless steel and having an appropriate wand thickness.

Directional control valves are used in fluid technology (pneumatics and hydraulics) to block the path for the working medium (compressed gas, hydraulic fluid) or to change the direction of flow.

A deposit in the sense of the present invention may be a gas storage facility e.g. for gas, especially for natural gas, but also for hydrogen and petroleum, carbon dioxide or even other gas or mixed gas. Also, the deposit may be a deposit for liquid raw materials, such as oil. The term deposit may further mean in the sense of the present invention a natural or artificial cavity, tank or even an infrastructure (e.g. piping) comprising the fluid of raw materials (gas and/or liquid) stored in its inner part. The deposit may be above ground or below the surface of the earth. Preferably, in the deposit, the raw material is stored above 10 bar and up to 200 bar pressure or even more such as 250 bar. The deposit may be a natural gas storage facility. Such facilities are large, and mostly underground and can be used to compensate for seasonal fluctuations in demand and supply shortages of e.g. natural gas. An underground deposit in the sense of the invention may mean a term from applied geology and mining and means certain areas of the earth's crust in which there are natural concentrations of solid, liquid or gaseous raw materials whose extraction is economically worthwhile (buildable deposit), or could be worthwhile in the future (usable deposit). Such underground deposits may also be caverns build in a mountain or even undersea deposits. Especially, such underground deposit may be oil and natural gas deposits. Since the underground deposits are in the inner of the earth, they normally exhibit in their inner high pressures over 10 bar and high temperatures over 40° C or even over 80° C and more.

When, according to the present invention, there is mention of the fact that a sample is taken from an underground deposit, it is meant that the sample may also be taken indirectly from an infrastructure coupled to the underground deposit. That means that the infrastructure can be above ground, but is connected to the underground deposit in a flow-conducting manner. To the infrastructure may belong: i.e. pipelines, valves and gas treatment plants.

According to an embodiment, the directional control valve is designed as a ball valve. Thus, a mechanically simple, inexpensive and reliable component can be achieved.

Preferably, a shut-off valve is associated with the outlet and is connected downstream of the outlet in the direction of flow of the raw material sample out of the outlet, wherein the shut-off valve is movable between an open position, in which it completely opens the flow cross-section for the raw material sample from the outlet, and a closed position, in which it completely closes the flow cross-section, and is preferably designed as a needle valve. In this way, the sample chamber can be slowly decompressed from the pressure in the deposit down to the pressure needed for examination of the raw material samples. Thus, a sudden cell death of the anaerobic microorganisms can be largely avoided.

The high-pressure container may at least comprise one or more pressure safety devices, such as a pressure relief valve or bursting disc, which is associated with the high-pressure container, which pressure safety device is connected to the sample chamber in a flow-conducting manner and the pressure safety device is designed in such a way that it triggers, preferably above 150 bar, preferably above 170 bar and most preferably above 199 bar, in order to reduce an excess pressure in the sample chamber. This enables safe operation of the high-pressure container e.g. in case of the occurrence of a pressure peak in the deposit.

In order to control or measure the pressure in the sample chamber, at least one or more pressure manometers are associated with the high-pressure container and are connected to the sample chamber in a flow-conducting manner.

To enhance the sample collection out of the sample chamber, a septum may be associated with the high-pressure container, which septum is connected to the sample chamber in order to remove at least a part of the raw material sample from the sample chamber via the septum. A shut-off valve, such as a ball valve, can be arranged between the sample chamber and the septum to protect the septum from excessive pressures in the sample chamber. Therefore, sampling through the septum can take place as soon as the pressure in the sample chamber has been reduced e.g. through the outlet to a lower pressure level, something between 1 and 5 bar. Thus, a convenient and easy way of getting access to the raw material sample stored in the sample chamber is possible.

The high-pressure container comprises a sample container and a lid which can be placed thereon, which together delimit or form the sample chamber, wherein preferably the lid forms the inlet and the outlet for the raw material sample and the feed line and the directional control valve are fastened or can be fastened to the lid, preferably in the embodiment according to claim 4, the pressure safety device is fastened to the lid and preferably in the embodiment according to claim 5, the pressure manometer is fastened or can be fastened to the lid. Thus, a compact high-pressure container can be provided that in whole can be easily transported by hand to and from the sampling point or the deposit and as well be completely sterilised in an autoclave. For this case, the pressure manometer(s) can be detachably fastened to and from the lid.

Preferably, the dimensions of the high-pressure container may be chosen that way that it fits in a conventional sluice of an anaerobic chamber such as an anaerobic work tent, Type B, 7-2651 from Coy Laboratory Products Inc.

The present invention also concerns a method for taking a raw material sample containing oxygen-sensitive, such as anaerobic, microorganisms from a deposit, preferably a gas deposit, by means of a high-pressure container, comprising the following steps:
a) connecting the high-pressure container, the sample chamber of which is sterilised and evacuated or filled with inert gas to receive the raw material sample, preferably pressurised with overpressure;
b) flushing the feed line with the raw material sample while bypassing the sample chamber;
c) feeding the raw material sample via the flushed feed line and an inlet into the sample chamber of the high-pressure container for filling the same.

Preferably, in the method, the step c) may be followed by the following steps:
d) disconnecting the high-pressure container from the deposit;
e) placing the high-pressure container in a protective atmosphere for examining the raw material sample;
f) reducing the pressure in the sample chamber to a pressure above or equal to the pressure of the protective atmosphere;
g) taking the sample from the sample chamber.

Thereby, the high-pressure container used in the inventive method may be the inventive high-pressure container, wherein preferably in step b) of the method the directional control valve of the high-pressure container is brought into such a position that by means of the directional control valve the feed line can be connected to an outlet for the raw material sample, bypassing the inlet, in order to flush the feed line with the raw material sample and preferably in step c) the directional control valve is brought into such a position, that the feed line can be connected to the inlet in a flow-conducting manner in order to feed the raw material sample from the feed line via the inlet to the sample chamber, preferably the high-pressure container being designed according to claim 3, so that in step f) the shut-off valve associated with the outlet is brought in the direction of its open position, in which it releases the flow cross-section for the raw material sample from the outlet, in order to reduce the pressure in the sample chamber to a pressure above or equal to the pressure of the protective atmosphere.

The advantages of the invention will now be illustrated in more detail with reference to a preferred embodiment and the figures, showing:
- Fig. 1: a schematic, spatial view on a hig-pressure container according to an embodiment of the present invention;
- Fig. 2: a vertical cut through the high-pressure container as shown in Fig. 1.

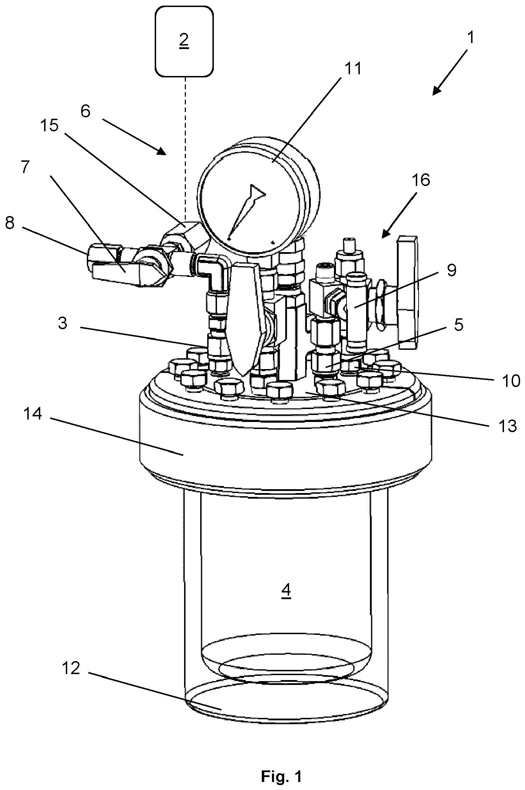

Fig. 1 discloses a schematic, spatial view on a high-pressure container 1 according to an embodiment of the present invention. The high-pressure container 1 is designed for taking a raw material sample containing oxygen-sensitive, such as anaerobic, microorganisms from a deposit 2, shown here schematically in dashed lines. In the deposit 2, pressures highly above of 10 bar exist.

The high-pressure container 1 comprises an inlet 3 for feeding the raw material sample under pressure into a sample chamber 4 of the high-pressure container 1 and an outlet 5 for discharging the raw material sample from the sample chamber 4, the inlet 3 being connectable to the deposit 2 by means of a feed line 6 and a directional control valve 7 being assigned to the inlet 3 and being connectable to the feed line 6. In detail, the feed line 6 can be connected to a port 15 in order to provide the sample raw material into the inlet 3. The control valve 7 being arranged upstream of the inlet 3 as seen in the direction of flow of the raw material sample from the deposit 2 into the sample chamber 4. As can best be seen from Fig. 1, the directional control valve 7 is arranged in between the port 15 and the inlet 3.

With the directional control valve 7, the feed line 6 can be selectively connected on the one hand to a discharge 8 for the raw material sample, bypassing the inlet 3, in order to flush the feed line 6 with the raw material sample, e.g. to the atmosphere. And on the other hand, the feed line 6 can be connected to the inlet 3 in a flow-conducting manner in order to feed the raw material sample from the feed line 6 via the inlet 3 to the sample chamber 4. Thereby, the directional control valve 7 can be designed as a ball valve.

Further, a shut-off valve 9 is associated with the outlet 5 and is connected downstream of the outlet 5 as seen in the direction of flow of the raw material sample, wherein the shut-off valve 9 is movable between an open position, in which it completely opens the flow cross-section for the raw material sample out from the sample chamber 4 passing the outlet 5, and a closed position, in which it completely closes such a flow cross-section. With the shut-off valve 9, which may be preferably designed as a needle valve, a controlled and slow decompression of the sample chamber 4 may be achieved.

To omit safety risks arising from pressure peaks in the deposit when taking samples, the high-pressure container may comprise at least one pressure safety device 10, such as a pressure relief valve or a bursting disc. This pressure safety device 10 may then be associated with the high-pressure container 1, especially the sample chamber 4 in a flow-conducting manner. The pressure safety device 10 may be designed in such a way that it triggers, preferably above 150 bar, in order to reduce an excess pressure in the sample chamber 4.

In order to measure the pressure in the sample chamber 4, the high-pressure container 1 may comprise at least one pressure manometer 11 which may be connected to the sample chamber 4 in a flow-conducting manner in order to measure the pressure in the sample chamber 4.

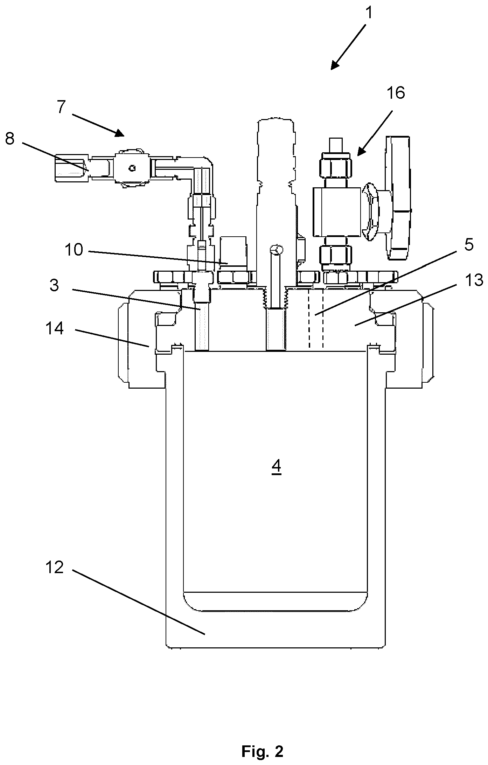

As can best be seen from Fig. 2, the high-pressure container 1 comprises a sample container 12 and a lid 13 which can be placed on the sample container 12, which sample container 12 and lid 13 together delimit or form the sample chamber 4. Both can be sealed pressure-tight against each other via a gasket. The sample container 12 and the lid 13 may be fastened together by the use of a ring 14, which can be bolted to the lid 13. The ring 14 may be made of two halves having a cross-section in the form of a "C" which encompass the lid 13 circumferentially.

Thereby, the lid 13 may form the inlet 3 and the outlet 5 for the raw material sample. Further, it may form or comprise fasteners with which the feed line 6 and the directional control valve 7 may be fastened to the lid 13. As shown, the pressure safety device 10 is also fastened to the lid 13 and also the pressure manometer 11 (Fig. 1). Thus, a very compact design of the high-pressure container 1 may be provided with all components fastened to it.

Once the sample was taken from the deposit 2 and e.g. brought to laboratory or an anaerobic tent, the sample from the sample chamber 4 may be taken from the outlet 5 by opening the shut-off valve 9 and pouring the sample out of the sample chamber 4, from the sample chamber 4 itself by opening the lid 13 or by a septum 16. In the last case, the septum 16 may be fluid-conducingly connected to the sample chamber 4 in order to remove at least a part of the raw material sample from the sample chamber 4. Theoretically, the sample could also be taken via the port 15 out of the sample chamber 4.

For taking the raw material sample containing oxygen-sensitive, such as anaerobic, microorganisms from the deposit 2, in a first step a) the high-pressure container 1 - the sample chamber 4 of which is sterilized and evacuated or filled with inert gas to receive the raw material sample - is connected to the deposit 2 by the feed line 6. This can be done by e.g. screwing or otherwise connecting the feed line 6 to a corresponding port 15 which can be brought in fluid-communication with the inlet 3 by the directional control valve 7. But first, the fluid-connection between deposit 2 and sample chamber 4 is cut off by the directional control valve 7.

In the following step b) the feed line 6 is flushed with the raw material sample while bypassing the sample chamber 4, thus flushing out any atmospheric oxygen that may still be present in the feed line 6 or the port 15. Thereby, the directional control valve 7 of the high-pressure container 1 is brought into such a position that by means of the directional control valve 7 the feed line 6 can be connected to a discharge 8 for the raw material sample, bypassing the inlet 3, in order to flush the feed line 6 with the raw material sample. Thus, the first parts of the raw material stemming from the deposit 2 are considered as scrap and thus are discarded.

In the next step c), the directional control valve 7 is brought into such a position, that the feed line 6 can be connected via the port 15 and the directional control valve 7 to the inlet 3 in a flow-conducting manner in order to feed the raw material sample from the feed line 6 via the inlet 3 to the sample chamber 4. Thus, the raw material sample is fed via the flushed feed line 6 and the inlet 3 into the sample chamber 4 of the high-pressure container 1, thus filling the same and gathering the sample without any atmospheric oxygen coming into the sample chamber 4.

In order to allow the protective gas contained in the sample chamber 4 to flow out, the shut-off valve 9 may be opened such that the gas can escape through the outlet 5 to the atmosphere.

After the high-pressure container 1, in detail, the sample chamber 4, has been sufficiently filled, the directional control valve 7 may be actuated such that a flow of the raw material sample from the deposit 2 into the inlet 3 is stopped. The correct filling level can be checked by means of the pressure manometer 11 and/or a scale on which the high-pressure container 1 can be placed. An eventually opened shut-off valve 9 may now be closed such that no raw material escapes from the sample chamber 4.

After that, in the following step d) the high-pressure container 1 is disconnected from the deposit 2. This can be done by disconnecting the feed line 6 from the port 15.

Then, in step e) the high-pressure container 1 is taken away and transported e.g. to a laboratory and placed in a protective atmosphere like an anaerobic work tent for examining the raw material sample. This could be done by placing the whole high-pressure container 1 in a conventional sluice of an anaerobic chamber of the anaerobic work tent.

In the following step f), the pressure in the sample chamber 4 is especially slowly reduced to a pressure above or equal to the pressure of the protective atmosphere, e.g. the atmosphere in the working tent. This can be done by the shut-off valve 9 associated with the outlet 5. By bringing the shut-off valve 9 in the direction of its open position, in which it releases the flow cross-section for the raw material sample from the outlet 5 to the atmosphere, the pressure in the sample chamber 4 can be reduced to a pressure above or equal to the pressure of the protective atmosphere.

Then, in the step g) the sample can be taken from the sample chamber 4. This can be done, as already disclosed by the outlet 5, the septum 16 or by opening the sample chamber 4 when releasing the lid 13 from the sample container 12.

With the present invention, a high-pressure container and a method for taking a raw material sample containing oxygen-sensitive, such as anaerobic, microorganisms from a deposit can be obtained that are improved as compared to the devices or methods known from the state of the art. In detail, the inventive high-pressure container as well as the sampling method provides a more reliable detection for anaerobic microorganisms, increases the survival rate of the sampled microorganisms as well as lowers the effort for sampling and cultivation of the microorganisms after taking the sample and examining it in the laboratory.

### Reference signs

- 1: high-pressure container
- 2: deposit
- 3: inlet
- 4: sample chamber
- 5: outlet
- 6: feed line
- 7: directional control valve
- 8: discharge
- 9: shut-off valve
- 10: pressure safety device
- 11: pressure manometer
- 12: sample container
- 13: lid
- 14: ring
- 15: port
- 16: septum

### Experimental section

### Experiments and results:

The inventive method for sampling of living microorganisms from anaerobic, high-pressure environments with the inventive high-pressure container 1 was tested on four different sampling sites E1S1, E1S2, E1S3 and B1S1 at two different gas storage facilities E1 and B1 (deposits 2). The sampling sites were located at different sections of the infrastructure containing either a liquid-gas mixture or liquid only. At the sampling sites, the operating pressure was between 63 and 154 bar. In addition to sampling with the inventive high-pressure container 1, samples were taken by the use of glass flasks. These glass flasks were previously flushed with nitrogen to achieve an oxygen-free atmosphere, closed with an airtight stopper and sterilized by heat (autoclaving). These flasks were opened just before sampling and held under the tap at the sampling point. The flasks were filled to the maximum and closed with an airtight stopper. The high-pressure container 1 was also sterilized by heat (autoclaving) and flushed with sterile nitrogen to achieve an oxygen-free atmosphere in the container before sampling. The high-pressure container 1 was connected to the sampling site by its feed line 6, which was sterilized by heat but which was not anaerobic. To achieve anaerobic conditions inside the feed line 6, it was flushed with liquid or liquid-gas mixture, respectively, from the sampling site, by the use of the directional control valve 7. Subsequently after discharging a part of the raw sample material through the directional control valve 7 and the discharge 8 in order to flush the feed line 6, the high-pressure container, in detail its sample chamber 4 was filled with the sample material from the deposit 2.

To compare the quality of high-pressure container 1 samples and flask samples both were analyzed for living microorganisms. In addition, the samples were analyzed for precipitated material, which can be an indication for chemical reactions of metal ions of the sample with oxygen that contaminated the sample during the sampling procedure. While the high-pressure container 1 sample had a clear liquid in it, the flask sample was turbid. The turbidity of the bottle sample came from a precipitate that settled after a while. Both, the high-pressure container 1 sample and the flask sample were analyzed for the amount of precipitate. Therefore, 120 ml of each sample was filtered by filtration crucibles (Porcelain C110, ceramic filter frit, 6 µm, VWR) and the crucibles with precipitation were dried at 100 °C for 1 hour. Weight comparison showed that the high-pressure container 1 sample contained 0.190 g/l precipitate while the bottle sample contained 0.364 g/l precipitate. This demonstrates that the sampling method with a high-pressure container according to the present invention prevented precipitation of chemical compounds. Precipitation is a consequence of chemical reactions, which are in this case prevented or at least significantly reduced by the high-pressure container 1.

Analysis of living microorganisms was conducted with the Water-Glo^{™} Microbial Water Testing Kit (Promega). This system enables the detection of adenosine 5'-triphosphate (ATP) which is a universal biomolecule present in any living cell. ATP is the major energy currency of cells and is involved in many cellular processes. Therefore, ATP concentrations are a measure for viable biomass. With this system high-pressure container 1 samples and flask samples from the above four different sampling sites at two different gas storage facilities were also analyzed. Tab. 1 shows the results of these measurements. At all four sampling sites the ATP concentration in samples from the high-pressure container were higher compared to the flask samples indicating that the high-pressure container 1 samples contained more living microorganisms. While at sampling site E1S2 two times more ATP was detected in the high-pressure container 1 sample, at sampling site E1S1 even 17-times more ATP was detected. As the sampling sites represent different parts of the infrastructure of the gas storage facilities it is very likely that different microbial strains or communities colonize these locations. It can be expected that some of these microbes might be more, others might be less sensitive to oxygen or rapid decompression.

**Tab. 1**

| Sampling site | Pressure at sampling site [bar] | Sample type | ATP concentration [pg/mL] |
|---|---|---|---|
| E1S1 | 63 | flask | 0.08 |
| | | container | 1.41 |
| E1S2 | 154 | flask | 1.78 |
| | | container | 3.46 |
| E1S3 | 154 | flask | 0.16 |
| | | container | 0.49 |
| B1S1 | 60 | flask | 0.04 |
| | | container | 0.43 |

## Claims

1. High-pressure container (1) for taking a raw material sample containing oxygen-sensitive, such as anaerobic, microorganisms from a deposit (2), preferably a gas deposit, the high-pressure container (1) comprising an inlet (3) for feeding the raw material sample under pressure into a sample chamber (4) of the high-pressure container (1) and an outlet (5) for discharging the raw material sample therefrom, the inlet (3) being connectable to the deposit (2) by means of a feed line (6) and a directional control valve (7) being assigned to the feed line (6), by means of which the feed line (6) can be selectively connected on the one hand to a discharge (8) for the raw material sample, bypassing the inlet (3), in order to flush the feed line (6) with the raw material sample and on the other hand the feed line (6) can be connected to the inlet (3) in a flow-conducting manner in order to feed the raw material sample from the feed line (6) via the inlet (3) to the sample chamber (4), wherein the high-pressure container (1) comprises a sample container (12) and a lid (13) which can be placed thereon, which together delimit or form the sample chamber (4), wherein the lid (13) forms the inlet (3) and the outlet (5) for the raw material sample and the feed line (6) and the directional control valve (7) are fastened or can be fastened to the lid (13); and wherein the directional control valve (7) is designed as a three-way valve, which being switchable between three positions, namely a first position, in which the raw material from the deposit (2) being guided under pressure to the discharge (8), a second position, in which the raw material from the deposit (2) being guided under pressure to the sample chamber (4) and a third position, in which the raw material being trapped in the sample chamber (4) by cutting of both return connections of the raw material sample to the discharge (8) or the deposit (2).

2. High-pressure container (1) according to claim 1, **characterised in that** the directional control valve (7) is designed as a ball valve.

3. High-pressure container (1) according to one of the claims 1 or 2, **characterised in that** a shut-off valve (9) is associated with the outlet (5) and is connected downstream of the outlet (5) in the direction of flow of the raw material sample out of the outlet (5), wherein the shut-off valve (9) is movable between an open position, in which it completely opens the flow cross-section for the raw material sample from the outlet (5), and a closed position, in which it completely closes the flow cross-section, and is preferably designed as a needle valve.

4. High-pressure container (1) according to one of the claims 1 to 3, **characterised in that** at least one pressure safety device, such as a pressure relief valve or bursting disc, is associated with the high-pressure container (1), which pressure safety device (10) is connected to the sample chamber (4) in a flow-conducting manner and the pressure safety device (10) is designed in such a way that it triggers, preferably above 150 bar, in order to reduce an excess pressure in the sample chamber (4).

5. High-pressure container (1) according to one of claims 1 to 4, **characterised in that** at least one pressure manometer (11) is associated with the high-pressure container (1) and is connected to the sample chamber (4) in a flow-conducting manner in order to measure the pressure in the sample chamber (4).

6. High-pressure container (1) according to one of the claims 1 to 5, **characterised in that** a septum (16) is associated with the high-pressure container (1), which septum (16) is connected to the sample chamber (4) in order to remove at least a part of the raw material sample from the sample chamber (4) via the septum (16).

7. High-pressure container (1) according to one of the claims 1 to 6, **characterised in that** in the embodiment according to claim 4, the pressure safety device (10) is fastened to the lid (13) and preferably in the embodiment according to claim 5, the pressure manometer (11) is fastened or can be fastened to the lid (13).

8. Method for taking a raw material sample containing oxygen-sensitive, such as anaerobic, microorganisms from a deposit (2), preferably a gas deposit, by means of a high-pressure container (1), comprising the following steps:
a) Connecting the high-pressure container (1), the sample chamber (4) of which is sterilised and evacuated or filled with inert gas to receive the raw material sample, preferably pressurised with overpressure to the deposit (2);
b) Flushing the feed line (6) with the raw material sample while bypassing the sample chamber (4);
c) feeding the raw material sample via the flushed feed line (6) and an inlet (3) into the sample chamber (4) of the high-pressure container (1) for filling the same.

9. Method according to claim 8, **characterized in that** step c) is followed by the following steps:
d) disconnecting the high-pressure container (1) from the deposit (2);
e) placing the high-pressure container (1) in a protective atmosphere for examining the raw material sample;
f) reducing the pressure in the sample chamber (4) to a pressure above or equal to the pressure of the protective atmosphere;
g) taking the sample from the sample chamber (4).

10. Method according to claim 8 or 9, wherein the high-pressure container (1) used is the high-pressure container (1) according to any one of claims 1 to 8, wherein preferably in step b) of the method the directional control valve (7) of the high-pressure container (1) is brought into such a position that by means of the directional control valve (7) the feed line (6) can be connected to an outlet (5) for the raw material sample, bypassing the inlet (3), in order to flush the feed line (6) with the raw material sample and preferably in step c) the directional control valve (7) is brought into such a position, that the feed line (6) can be connected to the inlet (3) in a flow-conducting manner in order to feed the raw material sample from the feed line (6) via the inlet (3) to the sample chamber (4), preferably the high-pressure container (1) being designed according to claim 3, so that in step f) the shut-off valve (9) associated with the outlet (5) is brought in the direction of its open position, in which it releases the flow cross-section for the raw material sample from the outlet (5), in order to reduce the pressure in the sample chamber (4) to a pressure above or equal to the pressure of the protective atmosphere.

## Patentansprüche

1. Hochdruckbehälter (1) zum Entnehmen einer sauerstoffempfindliche, wie etwa anaerobe, Mikroorganismen enthaltenden Rohstoffprobe aus einer Lagerstätte (2), vorzugsweise einer Gaslagerstätte, wobei der Hochdruckbehälter (1) einen Einlass (3) zum Zuführen der unter Druck stehenden Rohstoffprobe in eine Probenkammer (4) des Hochdruckbehälters (1) und einen Auslass (5) zum Austragen der Rohstoffprobe aus dieser umfasst, wobei der Einlass (3) mittels einer Zuleitung (6) mit der Lagerstätte (2) verbindbar ist und der Zuleitung (6) ein Wegeventil (7) zugeordnet ist, mittels dessen die Zuleitung (6) wahlweise einerseits mit einem den Einlass (3) umgehenden Austrag (8) für die Rohstoffprobe verbunden werden kann, um die Zuführleitung (6) mit der Rohstoffprobe zu spülen, und andererseits die Zuführleitung (6) mit dem Einlass (3) strömungsleitend verbunden werden kann, um die Rohstoffprobe von der Zuleitung (6) über den Einlass (3) in die Probenkammer (4) zuzuführen, wobei der Hochdruckbehälter (1) einen Probenbehälter (12) und einen darauf aufsetzbaren Deckel (13) umfasst, die gemeinsam die Probenkammer (4) begrenzen oder bilden, wobei der Deckel (13) den Einlass (3) und den Auslass (5) für die Rohstoffprobe bildet und die Zuleitung (6) und das Wegeventil (7) an dem Deckel (13) befestigt oder befestigbar sind; und wobei das Wegeventil (7) als ein Dreiwegeventil ausgebildet ist, das zwischen drei Stellungen umschaltbar ist, nämlich einer ersten Stellung, in der der Rohstoff aus der Lagerstätte (2) unter Druck zum Austrag (8) geführt wird, einer zweiten Stellung, in der der Rohstoff aus der Lagerstätte (2) unter Druck zu der Probenkammer (4) geführt wird, und einer dritten Stellung, in der der Rohstoff durch Absperren beider Rückführverbindungen der Rohstoffprobe zu dem Austrag (8) oder zu der Lagerstätte (2) in der Probenkammer (4) eingeschlossen wird.

2. Hochdruckbehälter (1) nach Anspruch 1, **dadurch gekennzeichnet, dass** das Wegeventil (7) als Kugelventil ausgebildet ist.

3. Hochdruckbehälter (1) nach einem der Ansprüche 1 oder 2, **dadurch gekennzeichnet, dass** dem Auslass (5) ein Absperrventil (9) zugeordnet ist, das in Strömungsrichtung der Rohstoffprobe aus dem Auslass (5) stromabwärts des Auslasses (5) verbunden ist, wobei das Absperrventil (9) zwischen einer Offenstellung, in der es den Strömungsquerschnitt für die Rohstoffprobe vom Auslass (5) vollständig freigibt, und einer Schließstellung, in der es den Strömungsquerschnitt vollständig verschließt, bewegbar ist und vorzugsweise als Nadelventil ausgebildet ist.

4. Hochdruckbehälter (1) nach einem der Ansprüche 1 bis 3, **dadurch gekennzeichnet, dass** dem Hochdruckbehälter (1) mindestens eine Drucksicherungsvorrichtung, wie etwa ein Überdruckventil oder eine Berstscheibe, zugeordnet ist, wobei die Drucksicherungsvorrichtung (10) mit der Probenkammer (4) strömungsleitend verbunden ist und die Drucksicherungsvorrichtung (10) derart ausgebildet ist, dass sie vorzugsweise oberhalb 150 bar auslöst, um einen Überdruck in der Probenkammer (4) abzusenken.

5. Hochdruckbehälter (1) nach einem der Ansprüche 1 bis 4, **dadurch gekennzeichnet, dass** dem Hochdruckbehälter (1) mindestens ein Druckmanometer (11) zugeordnet ist, das zum Messen des Drucks in der Probenkammer (4) strömungsleitend mit der Probenkammer (4) verbunden ist.

6. Hochdruckbehälter (1) nach einem der Ansprüche 1 bis 5, **dadurch gekennzeichnet, dass** dem Hochdruckbehälter (1) ein Septum (16) zugeordnet ist, wobei das Septum (16) mit der Probenkammer (4) verbunden ist, um mindestens einen Teil der Rohstoffprobe aus der Probenkammer (4) über das Septum (16) zu entnehmen.

7. Hochdruckbehälter (1) nach einem der Ansprüche 1 bis 6, **dadurch gekennzeichnet, dass** bei der Ausführungsform nach Anspruch 4 die Drucksicherungsvorrichtung (10) an dem Deckel (13) befestigt ist und bei der Ausführungsform nach Anspruch 5 das Druckmanometer (11) vorzugsweise am Deckel (13) befestigt ist oder befestigbar ist.

8. Verfahren zum Entnehmen einer sauerstoffempfindliche, wie etwa anaerobe, Mikroorganismen enthaltenden Rohstoffprobe aus einer Lagerstätte (2), vorzugsweise einer Gaslagerstätte, mittels eines Hochdruckbehälters (1), umfassend die folgenden Schritte:
a) Verbinden des Hochdruckbehälters (1), dessen Probenkammer (4) zum Aufnehmen der Rohstoffprobe sterilisiert und evakuiert oder mit Inertgas gefüllt, vorzugsweise mit Überdruck gegenüber der Lagerstätte (2) beaufschlagt ist;
b) Spülen der Zuleitung (6) mit der Rohstoffprobe unter Umgehen der Probenkammer (4);
c) Zuführen der Rohstoffprobe über die gespülte Zuleitung (6) und einen Einlass (3) in die Probenkammer (4) des Hochdruckbehälters (1) zum Befüllen desselben.

9. Verfahren nach Anspruch 8, **dadurch gekennzeichnet, dass** dem Schritt c) die folgenden Schritte folgen:
d) Abtrennen des Hochdruckbehälters (1) von der Lagerstätte (2);
e) Einbringen des Hochdruckbehälters (1) in eine Schutzatmosphäre zum Untersuchen der Rohstoffprobe;
f) Absenken des Drucks in der Probenkammer (4) auf einen Druck oberhalb oder gleich dem Druck der Schutzatmosphäre;
g) Entnehmen der Probe aus der Probenkammer (4).

10. Verfahren nach Anspruch 8 oder 9, wobei der verwendete Hochdruckbehälter (1) der Hochdruckbehälter (1) nach einem der Ansprüche 1 bis 8 ist, wobei vorzugsweise in Schritt b) des Verfahrens das Wegeventil (7) des Hochdruckbehälters (1) in eine solche Stellung gebracht wird, dass die Zuleitung (6) unter Umgehen des Einlasses (3) mit einem Auslass (5) für die Rohstoffprobe mittels des Wegeventils (7) verbindbar ist, um die Zuleitung (6) mit der Rohstoffprobe zu spülen, und vorzugsweise in Schritt c) das Wegeventil (7) in eine solche Stellung gebracht wird, dass die Zuleitung (6) mit dem Einlass (3) strömungsleitend verbunden werden kann, um die Rohstoffprobe von der Zuleitung (6) über den Einlass (3) der Probenkammer (4) zuzuführen, wobei vorzugsweise der Hochdruckbehälter (1) nach Anspruch 3 ausgebildet ist, sodass in Schritt f) das dem Auslass (5) zugeordnete Absperrventil (9) in Richtung seiner Offenstellung gebracht wird, in der es den Strömungsquerschnitt für die Rohstoffprobe von dem Auslass (5) freigibt, um den Druck in der Probenkammer (4) auf einen Druck oberhalb oder gleich dem Druck der Schutzatmosphäre abzusenken.

## Revendications

1. Récipient à haute pression (1) pour le prélèvement d'un échantillon de matière première contenant des micro-organismes sensibles à l'oxygène, tels que des micro-organismes anaérobies, dans un dépôt (2), de préférence un dépôt gazeux, le récipient à haute pression (1) comprenant une entrée (3) pour acheminer l'échantillon de matière première sous pression dans une chambre d'échantillonnage (4) du récipient à haute pression (1) et une sortie (5) pour évacuer l'échantillon de matière première de celui-ci, l'entrée (3) pouvant être reliée au dépôt (2) au moyen d'une conduite d'alimentation (6) et une soupape de commande directionnelle (7) étant attribuée à la conduite d'alimentation (6), au moyen de laquelle la conduite d'alimentation (6) peut être reliée sélectivement d'une part à une évacuation (8) pour l'échantillon de matière première, en contournant l'entrée (3), afin de rincer la conduite d'alimentation (6) avec l'échantillon de matière première et d'autre part la conduite d'alimentation (6) peut être reliée à l'entrée (3) de manière à conduire l'écoulement afin d'acheminer l'échantillon de matière première de la conduite d'alimentation (6) via l'entrée (3) vers la chambre d'échantillonnage (4), dans lequel le récipient à haute pression (1) comprend un récipient d'échantillon (12) et un couvercle (13) qui peut être placé sur celui-ci, lesquels ensemble délimitent ou forment la chambre d'échantillonnage (4), dans lequel le couvercle (13) forme l'entrée (3) et la sortie (5) pour l'échantillon de matière première et la conduite d'alimentation (6) et la soupape de commande directionnelle (7) sont fixées ou peuvent être fixées au couvercle (13) ; et dans lequel la soupape de commande directionnelle (7) est conçue sous la forme d'une soupape à trois voies, qui peut être commutée entre trois positions, à savoir une première position, dans laquelle la matière première provenant du dépôt (2) est guidée sous pression vers l'évacuation (8), une deuxième position, dans laquelle la matière première provenant du dépôt (2) est guidée sous pression vers la chambre d'échantillonnage (4) et une troisième position, dans laquelle la matière première est piégée dans la chambre d'échantillonnage (4) en coupant les deux raccords de retour de l'échantillon de matière première vers l'évacuation (8) ou le dépôt (2).

2. Récipient à haute pression (1) selon la revendication 1, **caractérisé en ce que** la soupape de commande directionnelle (7) est conçue sous la forme d'une soupape à bille.

3. Récipient à haute pression (1) selon l'une des revendications 1 ou 2, **caractérisé en ce qu'**une soupape d'arrêt (9) est associée à la sortie (5) et est reliée en aval de la sortie (5) dans le sens d'écoulement de l'échantillon de matière première hors de la sortie (5), dans lequel la soupape d'arrêt (9) est mobile entre une position ouverte, dans laquelle elle ouvre complètement la section transversale d'écoulement de l'échantillon de matière première depuis la sortie (5), et une position fermée, dans laquelle elle ferme complètement la section transversale d'écoulement, et est de préférence conçue sous la forme d'une soupape à aiguille.

4. Récipient à haute pression (1) selon l'une des revendications 1 à 3, **caractérisé en ce qu'**au moins un dispositif de sûreté haute pression, tel qu'une soupape de sûreté ou un disque de rupture, est associé au récipient à haute pression (1), lequel dispositif de sûreté haute pression (10) est relié à la chambre d'échantillonnage (4) de manière à conduire l'écoulement et le dispositif de sûreté haute pression (10) est conçu de telle sorte qu'il se déclenche, de préférence au-dessus de 150 bar, afin de réduire une surpression dans la chambre d'échantillonnage (4).

5. Récipient à haute pression (1) selon l'une des revendications 1 à 4, **caractérisé en ce qu'**au moins un manomètre de pression (11) est associé au récipient à haute pression (1) et est relié à la chambre d'échantillonnage (4) de manière à conduire l'écoulement afin de mesurer la pression dans la chambre d'échantillonnage (4).

6. Récipient à haute pression (1) selon l'une des revendications 1 à 5, **caractérisé en ce qu'**un septum (16) est associé au récipient à haute pression (1), lequel septum (16) est relié à la chambre d'échantillonnage (4) afin de retirer au moins une partie de l'échantillon de matière première de la chambre d'échantillonnage (4) via le septum (16).

7. Récipient à haute pression (1) selon l'une des revendications 1 à 6, **caractérisé en ce que** dans le mode de réalisation selon la revendication 4, le dispositif de sûreté haute pression (10) est fixé au couvercle (13) et de préférence dans le mode de réalisation selon la revendication 5, le manomètre de pression (11) est fixé ou peut être fixé au couvercle (13).

8. Procédé de prélèvement d'un échantillon de matière première contenant des micro-organismes sensibles à l'oxygène, tels que des micro-organismes anaérobies, dans un dépôt (2), de préférence un dépôt gazeux, au moyen d'un récipient à haute pression (1), comprenant les étapes suivantes :
a) raccordement du récipient à haute pression (1) dont la chambre d'échantillonnage (4) est stérilisée et vidée ou remplie de gaz inerte pour recevoir l'échantillon de matière première, de préférence pressurisé en surpression au dépôt (2) ;
b) rinçage de la conduite d'alimentation (6) avec l'échantillon de matière première tout en contournant la chambre d'échantillonnage (4) ;
c) acheminement de l'échantillon de matière première via la conduite d'alimentation rincée (6) et une entrée (3) dans la chambre d'échantillonnage (4) du récipient à haute pression (1) pour le remplir.

9. Procédé selon la revendication 8, **caractérisé en ce que** l'étape c) est suivie des étapes suivantes :
d) débranchement du récipient à haute pression (1) du dépôt (2) ;
e) placement du récipient à haute pression (1) dans une atmosphère protectrice pour l'examen de l'échantillon de matière première ;
f) réduction de la pression dans la chambre d'échantillonnage (4) à une pression supérieure ou égale à la pression de l'atmosphère protectrice ;
g) prélèvement de l'échantillon dans la chambre d'échantillonnage (4).

10. Procédé selon la revendication 8 ou 9, dans lequel le récipient à haute pression (1) utilisé est le récipient à haute pression (1) selon l'une quelconque des revendications 1 à 8, dans lequel de préférence à l'étape b) du procédé, la soupape de commande directionnelle (7) du récipient à haute pression (1) est amenée dans une position telle que, au moyen de la soupape de commande directionnelle (7), la conduite d'alimentation (6) peut être reliée à une sortie (5) pour l'échantillon de matière première, en contournant l'entrée (3), afin de rincer la conduite d'alimentation (6) avec l'échantillon de matière première et de préférence à l'étape c), la soupape de commande directionnelle (7) est amenée dans une position telle que la conduite d'alimentation (6) peut être reliée à l'entrée (3) de manière à conduire l'écoulement afin d'acheminer l'échantillon de matière première de la conduite d'alimentation (6) via l'entrée (3) vers la chambre d'échantillonnage (4), de préférence le récipient à haute pression (1) est conçu selon la revendication 3, de sorte qu'à l'étape f) la soupape d'arrêt (9) associée à la sortie (5) est amenée dans la direction de sa position ouverte, dans laquelle elle libère la section transversale d'écoulement pour l'échantillon de matière première depuis la sortie (5), afin de réduire la pression dans la chambre d'échantillonnage (4) à une pression supérieure ou égale à la pression de l'atmosphère protectrice.
